# NEW EUROPEAN PATENT SPECIFICATION

(11) **EP 1 404 385 B2**
(45) Date of publication and mention of the opposition decision: **06.07.2011**
(45) Mention of the grant of the patent: 17.01.2007
(21) Application number: 02756355.0
(22) Date of filing: 26.06.2002
(51) Int. Cl.: A61L 15/60, A61L 15/46, A61L 15/20, A61L 15/18

(54) **SUPERABSORBENT CARBOXYL-CONTAINING POLYMERS WITH ODOR CONTROL**
SUPERABSORBIERENDE CARBOXYL ENTHALTENDE POLYMERE MIT GERUCHSKONTROLLE
POLYMERES SUPERABSORBANTS CONTENANT UN CARBOXYLE ET PRESENTANT DES PROPRIETES D'ELIMINATION DES ODEURS, ET PROCEDE DE PREPARATION ASSOCIE

(30) Priority: 29.06.2001 US 302329 P
(43) Date of publication of application: 07.04.2004
(73) Proprietor: Evonik Stockhausen GmbH, 47805 Krefeld (DE)
(72) Inventor: KIM, Young-Sam, 77815 Buehl (DE)
(74) Representative: Herzog, Martin
(86) International application number: PCT/US2002/020874
(87) International publication number: WO 2003/002164

(56) References cited:
- EP-A- 0 475 807
- EP-A1- 0 291 587
- EP-A1- 0 745 393
- WO-A-00/46260
- WO-A-96/13282
- WO-A-98/06260
- WO-A-98/26808
- WO-A-99/01166
- WO-A1-00/22017
- WO-A1-00/78281
- WO-A1-01/41819
- WO-A1-91/11206
- WO-A1-99/03344
- DE-A1- 3 228 851
- JP-A- 4 092 664
- JP-A- 5 212 094
- US-A- 2 785 106
- US-A- 3 092 552
- US-A- 6 087 549
- US-B1- 6 180 132
- US-B1- 6 201 164
- DATABASE WPI Section Ch, Week 199047, Derwent Publications Ltd., London, GB; Class A96, AN 1990-351890, XP002320808 & JP 02 253847 A (DAINIPPON PRINTING CO LTD) 12 October 1990
- PATENT ABSTRACTS OF JAPAN vol. 016, no. 320 (C-0962) 14 July 1992 & JP 04 092664 A (ISHIZUKA GLASS CO LTD) 25 March 1992
- DATABASE WPI Section Ch, Week 199605, Derwent Publications Ltd., London, GB; Class D22, AN 1996-044093, XP002320809 & JP 07 308340 A (MATSUSHITA DENKI SANGYO KK) 28 November 1995
- PATENT ABSTRACTS OF JAPAN vol. 017, no. 655 (C-1136) 06 December 1993 & JP 05 212094 A (TORAY IND INC) 24 August 1993
- DATABASE WPI Section Ch, Week 197918, Derwent Publications Ltd., London, GB; Class A94, AN 1979-34307B, XP002320810 & JP 54 038951 A (TORAY IND INC) 24 March 1979
- PATENT ABSTRACTS OF JAPAN vol. 017, no. 547 (C-1116) 04 October 1993 & JP 05 154174 A 22 June 1993
- DATABASE WPI Section Ch, Week 199330, Derwent Publications Ltd., London, GB; Class A96, AN 1993-239286, XP002321529 & JP 05 191671 A (KANEBO LTD) 29 June 1993
- PATENT ABSTRACTS OF JAPAN vol. 1996, no. 09 30 September 1996 & JP 08 126659 A (TERUMO CORP) 21 May 1996
- PATENT ABSTRACTS OF JAPAN vol. 2000, no. 04 31 August 2000 & JP 2000 017571 A (KANEBO LTD; KANEBO SYNTHETIC FIBRES LTD) 18 January 2000
- DATABASE EMBASE [Online] ELSEVIER SCINECE PUBLISHERS, AMSTEDAM NL; 1999 MIYASATO K ET AL: 'A newly developed wound dressing made of alginate fiber containing zeolite substituted by silver and zinc: Fundamental study', XP002244428 Database accession no. EMB-1999300698 & JAPANESE PHARMACOLOGY AND THERAPEUTICS 1999 vol. 27, no. 4, 1999, JAPAN, pages 177 - 203 ISSN: 0386-3603
- 'Noveau Traité de Chimie Minérale', part III 1957 article PAUL PASCAL: 'Traité de chemie minérale', page 473
- KIRK-OTHMER: 'Encyclopedia of Chemial Technology - Third Edition', vol. 21, 1983 page 16

## Description

This invention relates to superabsorbent polymers with odor control properties.

Water-absorbent polymers, also referred to as superabsorbent polymers or aqueous fluid absorbent polymers, are primarily used in personal care products which absorb body fluids, for example, baby diapers, adult incontinence products and feminine hygiene products. In such applications, superabsorbent polymer particles are incorporated into absorbent structures which contain synthetic and/or natural fiber or paper based, woven and nonwoven structures, or toughened masses of fibers, such as fluff pads. The materials used in such structures can quickly absorb aqueous fluids and distribute them throughout the whole absorbent structure. The structures, in the absence of superabsorbent polymers, have limited absorption capacity, are bulky due to the large amount of material needed to provide acceptable absorption capacity, and do not retain fluid under pressure. A means for improving the absorbency and fluid retention characteristics of such absorbent structures is to incorporate superabsorbent polymer particles which imbibe fluids to form a swollen hydrogel material.

The superabsorbent polymer particles quickly absorb fluids and retain such fluids to prevent leakage and give the absorbent structure a "dry feel" even when wetted. See U.S. Patent 4,610,678 for examples of such polymers. See also U.S. Patents 4,654,039 and Re. 32,649, which disclose a process for the preparation of superabsorbent polymers and the use of known crosslinking agents for such polymers, and also U.S. Patents 4,295,987 and 4,303,771. A variation of the basic process is taught in GB Patent 2,119,384, which discloses a post polymerization surface crosslinking process in which the previously polymerized absorbent polymer powder is mixed with crosslinkers, preferably polyalcohols, a solvent and water, to coat the polymer surface and is heated to temperatures in the range of 90 to 300°C to crosslink the surface. U.S. Patent 5,506,324 discloses superabsorbent polymer particles comprising polymers containing carboxyl moieties which are crosslinked using C₂₋₁₀ polyhydric hydrocarbons which are ethoxylated with from 2 to 8 ethylene oxide units per hydroxyl moiety of the polyhydric hydrocarbon wherein the hydroxyl moiety at the end of each ethylene oxide chain is esterified with a C₂₋₁₀ unsaturated carboxylic acid or ester thereof. In a preferred embodiment, the superabsorbent polymer particles are subjected to a heat-treatment process after drying and sizing the particles.

Especially for the use of superabsorbent polymers in feminine hygiene products and adult incontinence products, it would be desirable to have a superabsorbent polymer that reduces unpleasant odors that might develop in use, particularly when contacted with bacteria infected urine. Different methods have been employed in the prior art to reduce malodor in superabsorbent polymer-containing devices.

Various odor-controlling agents are known in the prior art. Odors can be in general chemically classified as being basic, acidic and neutral. Odor-controlling agents can combat odors based on different mechanisms such as, for example, absorption, adsorption and inclusion complexation of malodor causing molecules, masking and modification of malodor causing molecules, inhibition of malodor producing micro-organisms or a combination of these mechanisms.

European Patent Publication 392 608 discloses a disposable absorbent polymer product which comprises a cyclodextrin, especially β-cyclodextrin, and an active agent, for example, a perfume. WO 99/64485 also relates to superabsorbent polymers containing cyclodextrins. However, cyclodextrins are biologically degradable, and are a good nurture for microorganisms. When contacted with microorganisms, such as the bacteria in infected urine, bacteria proliferation is increased, resulting in increased malodor. Furthermore, cyclodextrins are often very fine dusty substances which are difficult to handle on a large commercial processing scale.

U.S. Patent 4,385,632 is directed to an absorbent article for urine which contains a water-soluble copper salt, for example copper acetate, which impedes bacterial growth, prevents ammonia production and binds ammonia by complexation so as to prevent the occurrence of unpleasant odor. The copper ion treatment is less favorable not only due to its low efficacy even at relatively high concentrations in the case of heavy incontinence where severe urinary tract infection is present, but also due to coloring which may limit its use in hygiene articles from the aesthetic viewpoint.

U.S. Patent 6,096,299 discloses an absorbent article containing an odor control material that comprises a zeolite having a particle size of more than 200 µm. The zeolite may optionally be mixed with a superabsorbent polymer and activated carbon. WO 98/20915 concerns a superabsorbent composition containing a superabsorbent polymer powder and a zeolite powder exchanged with metal cations having bactericidal properties, such as Ag, Cu and Zn ions. It is a disadvantage of zeolite materials that they are less effective in controlling odor when used in swollen superabsorbent polymer gels. It is assumed that the odor absorbing capacity, that is, the pores of the zeolite, may partially be filled by water molecules instead of volatile odor-causing molecules. Furthermore, zeolite materials are in general fine dusty substances which are difficult to handle on a large commercial scale.

Japanese Patent Publication 05179053 relates to a method for producing a water absorbent polymer with good antimicrobial properties wherein the polymer contains a water-insoluble inorganic phosphate compound, for example, silver sodium hydrogen zirconium phosphate (sold under the tradename Antimicrobial ALPHASAN RC 5000 by Milliken Chemicals, USA). The inorganic phosphate compound has a general formula of M¹ₐA_{b}M²_{c}(PO₄)_{d}·nH₂O. M¹ is selected from Ag, Cu, Zn, Sn, Hg, Pb, Fe, Co, Ni, Mn, As, Sb, Bi, Ba, Cd and Cr. A is selected from alkali metal ions, alkaline earth metal ions, NH₄ and H, preferably, M¹ is, for example, Ag; A is, for example, Li, Na, NH₄ or H; M² is, for example, Zr, Ti or Sn. It is assumed that the M¹ ions captured in the network structure of the specified phosphate compound are released as in the case of heavy metal ion exchanged zeolites. However, these inorganic phosphate compounds have drawbacks similar to those of the zeolite materials mentioned above.

WO 00/78281 discloses an anti-microbial absorbent product comprising homogeneously dispersed particles of metallic silver having a particle size in the range of 1 to 50 nm. One embodiment relates to a disposable absorbent article comprising superabsorbent polymers. However, the preparation of the silver nano-particles is complicated.

As seen above, most existing methods are incapable of sufficiently reducing malodor, or have other drawbacks. They often require treatments with malodor adsorbents, or perfume/fragrance. The use of perfume/fragrance can mask the malodor but it can be difficult to match the personal odor preference of the user. Often, the offensiveness of the combination of malodor and perfume is perceived to be more than that of the malodor alone. The various treatments in the prior art involve complicated and time-consuming process steps and are also often detrimental with regard to the absorption capacity and other properties of the superabsorbent polymer. Therefore, it would be highly desirable to provide a superabsorbent polymer with odor control properties that is unaffected in its absorbency properties. It also would be desirable to develop a simple process for preparing the superabsorbent.

This invention relates to a process for the preparation of water-absorbent water-insoluble polymer particles which comprises:
(I) polymerizing a polymerization mixture comprising:
   (a) one or more ethylenically unsaturated carboxyl-containing monomers,
   (b) one or more crosslinking agents,
   (c) optionally one or more comonomers copolymerizable with the carboxyl-containing monomer, and
   (d) a polymerization medium, to form a crosslinked hydrogel,
(II) comminuting the hydrogel to particles, and (III) drying the hydrogel; wherein a solution of a silver salt which has a solubility in water at room temperature of not less than 1 g per liter is added in at least one of the following steps:
   (i) to the polymerization mixture prior to the beginning of the polymerization or to the reaction mixture during polymerization, or
   (ii) to the crosslinked hydrogel prior to or after comminution in step (11), or (iii) to the dried polymer particles after step (III).
Another aspect of the invention are polymer particles obtainable by the process of the invention. This invention also concerns an absorbent structure comprising the polymer particles of this invention and at least one of a woven or nonwoven structure of paper, synthetic fibers, or natural fibers.

The superabsorbent polymer of this invention is very effective in preventing malodor that can develop when a polymer comes in contact with biological fluids such as urine or blood. It is known that micro-organisms play an important role in the development of malodor. For example, bacteria strains that are capable of producing urease enzyme split the urea of the urine into ammonia and carbon dioxide. It is assumed that skin irritation, and the foul smell of urine, are mainly due to the production of ammonia by urea cleavage of urease from the bacteria in the urine and in the perineal region. Bacteria proliferation and ammonia production are significantly inhibited in a device comprising the superabsorbent polymers of the invention.

In principal, all metal ions may inactivate bacteria by reacting outside or inside the bacterial cell to some extent, either directly or indirectly. Indeed, various metal ions have been long known and used as antibacterial agents. It has now been found that silver ions show surprisingly improved positive odor control versus other antibacterial metal ions which might be commercially acceptable to diaper producers, such as aluminum, copper, and zinc.

It is indeed surprising that it is not necessary to use expensive and difficult to handle carriers like zeolites and specific insoluble inorganic phosphates in combination with silver ions to provide effective odor control. Thus, the silver cations in the present superabsorbent polymers are "free" ions, that is, they are neither included in zeolites nor bonded to phosphate anions in the form of insoluble phosphates.

The crucial point of the present invention is the presence of silver ions in the superabsorbent polymer, that is, the addition of a silver salt to the process of preparing the superabsorbent polymer.

The silver salts are applied to the superabsorbent polymer either in powdered salt form or as a solution or suspension. The solution can be aqueous, organic, or a mixture of these. Water-soluble silver salts, which are termed "soluble silver salts" in the present application, are the preferred source of silver ions. The solubility of diverse silver salts generally can be improved by acidifying them, dissolving them in alkalis, dissolving them in organic solvent, dissolving them at elevated temperatures, and/or intensive mixing during the dissolution process. The soluble silver salts have a solubility in water at room temperature of not less than 1 g per liter. Most preferably, the soluble silver salts have a solubility of not less than 10 g per liter.

Examples of silver salts include, for example silver acetate, silver benzoate, silver fluoride, silver bromate, silver chlorate, silver lactate, silver nitrate, silver nitrite, silver perchlorate, silver permanganate, silver selenate, and silver sulfate. Mixtures of various silver salts can also be used. The preferred silver salts are silver acetate, silver benzoate, silver bromate, silver chlorate, silver lactate, silver nitrate, silver nitrite, silver perchlorate, silver permanganate, silver selenate, and silver sulfate. The most preferred silver salts are silver acetate and silver nitrate. Mixtures of silver salts can be employed.

Advantageously, the amount of silver salt employed is such that the superabsorbent polymer preferably comprises silver cations in an amount of from 1 ppm to 10,000 ppm, more preferably from 1 to 3,000 ppm, even more preferably from 10 to 1,000 ppm and most preferably from 25 to 500 ppm, all based on the dry weight of the polymer. Preferably, amount of silver salt employed is at least 1 ppm, more preferably at least 10 ppm, and most preferably at least 25 ppm based on the weight of dry polymer. The amount of silver salt employed advantageously is at most 10,000 ppm, preferably at most 3,000 ppm, more preferably at most 1,000 ppm, and most preferably at most 500 ppm based on the weight of dry polymer.

The water-absorbent, water-insoluble polymers advantageously are derived from one or more ethylenically unsaturated carboxylic acids, ethylenically unsaturated carboxylic acid anhydrides or salts thereof. Additionally, the polymers may include comonomers known in the art for use in superabsorbent polymers or for grafting onto the superabsorbent polymers including comonomers such as an acrylamide, an acrylonitrile, a vinyl pyrrolidone, a vinyl sulphonic acid or a salt thereof, a cellulosic monomer, a modified cellulosic monomer, a polyvinyl alcohol or a starch hydrolyzate. If used, the comonomer comprises up to 25 percent by weight of the monomer mixture.

Preferred unsaturated carboxylic acid and carboxylic acid anhydride monomers include the acrylic acids typified by acrylic acid, methacrylic acid, ethacrylic acid, α-chloroacrylic acid, a -cyano acrylic acid, β-methyl acrylic acid (crotonic acid), α -phenyl acrylic acid, β-acryloyloxy propionic acid, sorbic acid, α-chloro sorbic acid, angelic acid, cinnamic acid, p-chloro cinnamic acid, beta-styrenic acrylic acid (1 -carboxy-4-phenyl butadiene-1,3), itaconic acid, citraconic acid, mesaconic acid, glutaconic acid, maleic acid, fumaric acid and maleic acid anhydride. More preferably the starting monomer is acrylic acid, methacrylic acid, or a salt thereof with acrylic acid or a salt thereof being most preferred. The use herein of the prefix "(meth)" with generic terms, such as, for example, "acrylic acid ", or "acrylate" is meant to broaden the terms to include both acrylate and methacrylate species. Thus, the term "(mcth)acrylic acid monomer" includes acrylic acid and methacrylic acid.

Preferably, 25 mole percent or greater of the carboxylic acid units of the hydrophilic polymer are neutralized with base, even more preferably 50 percent or greater and most preferably 65 percent or greater. This neutralization may be performed after completion of the polymerization. In a preferred embodiment the starting monomer mix has carboxylic acid moieties that arc neutralized to the desired level prior to polymerization. The final polymer or the starting monomers may be neutralized by contacting them with a salt forming cation. Such salt-forming cations include alkaline metal, ammonium, substituted ammonium and amine based cations. Preferably, the polymer is neutralized with an alkali metal hydroxide such as, for example, sodium hydroxide or potassium hydroxide, or an alkali metal carbonate such as, for example, sodium carbonate or potassium carbonate.

The water-absorbent polymers of the invention are lightly crosslinked to make them water-insoluble. Vinyl, non-vinyl, or dimodal crosslinkers can be employed, either alone, as mixtures, or in various combinations. Polyvinyl crosslinkers commonly known in the art for use in superabsorbent polymers advantageously are employed. Preferred compounds having at least two polymerizable double bonds include: di- or polyvinyl compounds such as divinyl benzene, divinyl toluene, divinyl xylene, divinyl ether, divinyl ketone and trivinyl benzene; di- or polyesters of unsaturated mono- or polycarboxylic acids with polyols, such as di- or tri-(meth)acrylic acid esters of polyols such as ethylene glycol, diethylene glycol, triethylene glycol, tetra ethylene glycol, propylene glycol, dipropylene glycol, tri propylene glycol, tetra propylene glycol, trimethylol propane, glycerin, polyoxyethylene glycols and polyoxypropylene glycols; unsaturated polyesters that can be obtained by reacting any of the above-mentioned polyols with an unsaturated acid such as maleic acid; di- or polyesters of unsaturated mono- or polycarboxylic acids with polyols derived from reaction of C₂-C₁₀ polyhydric alcohols with 2 to 8 C₂-C₄ alkylene oxide units per hydroxyl group, such as trimethylol propane hexaethoxyl triacrylate; di- or tri-(meth)acrylic acid esters that can be obtained by reacting polyepoxide with (meth)acrylic acid; bis(meth) acrylamides such as N,N-methylene-bisacrylamide; carbamyl esters that can be obtained by reacting polyisocyanates such as tolylene diisocyanate, hexamethylene diisocyanate, 4,4'-diphenyl methane diisocyanate and NCO-containing prepolymers obtained by reacting such diisocyanates with active hydrogen atom-containing compounds with hydroxyl group-containing monomers, such as di-(meth)acrylic acid carbamyl esters obtainable by reacting the above-mentioned diisocyanates with hydroxyethyl(meth)acrylate; di- or poly(meth)allyl ethers of polyols such as alkylene glycols, glycerol, polyalkylene glycols, polyoxyalkylene polyols and carbohydrates such as polyethylene glycol diallyl ether, allylated starch, and allylated cellulose; di- or poly-allyl esters of polycarboxylic acids, such as diallyl phthalate and diallyl adipate; and esters of unsaturated mono- or polycarboxylic acids with mono(meth)allyl ester of polyols, such as allyl methacrylate or (meth)acrylic acid ester of polyethylene glycol monoallyl ether.

The preferred classes of crosslinkers include, for example, bis(meth)acrylamides; allyl(meth)acrylates; di- or poly-esters of (meth)acrylic acid with polyols such as diethylene glycol diacrylate, trimethylol propane triacrylate, and polyethylene glycol diacrylate; and di- or polyesters of unsaturated mono- or poly-carboxylic acids with polyols derived from the reaction of C₁-C₁₀ polyhydric alcohols with 2 to 8 C₂-C₄ alkylene oxide units per hydroxyl group, such as ethoxylated trimethylol propane triacrylate. More preferably the crosslinking agents correspond to Formula 1:

R¹ ((̵R² O)̵ₙ-C(O)R³)ₓ Formula 1

wherein:
R¹ is a straight- or branched-chain polyalkoxy radical with 1 to 10 carbon atoms, optionally substituted with one or more oxygen atoms in the backbone, having x valences; R² is independently in each occurrence an alkylene group of 2 to 4 carbon atoms;
R³ is independently in each occurrence a straight- or branched-chain alkenyl moiety with 2 to 10 carbon atoms;
n is a number from 1 to 20; and
x is a number from 2 to 8.

In the most preferred embodiment the polyvinyl crosslinker corresponds to Formula 1 wherein R¹ is derived from trimethylolpropane, R² is ethylene -(CH₂CH₂)-, R³ is vinyl -(CH=CH₂), the average value of n is from 2 to 6, and x is 3. The most preferred polyvinyl crosslinker is highly ethoxylated trimethylolpropane triacrylate, containing an average of 15 to 16 ethoxyl groups per molecule of trimethylolpropane. Crosslinkers corresponding to Formula 1 are available from Craynor under the trademark Craynor and from Sartomer under the trademark Sartomer. Generally, the crosslinkers described by Formula 1 are found as a mixture of materials described by the formula and by-products resulting from the preparation process. Mixtures of polyvinyl crosslinkers can be employed.

The non-vinyl crosslinkers of this invention are agents having at least two functional groups capable of reacting with the carboxyl groups of the polymer, and include materials such as glycerin, polyglycols, ethylene glycol digylcidyl ether, and diamines. Many examples of these agents are given in U.S. Patents 4,666,983 and 4,734,478 which teach the application of such agents to the surface of absorbent polymer powder followed by heating to crosslink surface chains and improve absorption capacity and absorption rate. Additional examples are given in U.S. Patent 5,145,906 which teaches post-crosslinking with such agents. In the current invention, the non-vinyl crosslinkers advantageously are added homogeneously to the polymerization mixture at the start of the process. Preferred non-vinyl crosslinkers include hexane diamine, glycerin, ethylene glycol diglycidyl ether, ethylene glycol diacetate, polyethylene glycol 400, polyethylene glycol 600, and polyethylene glycol 1000. Examples of more preferred non-vinyl crosslinkers include polyethylene glycol 400 and polyethylene glycol 600. Mixtures of non-vinyl crosslinkers can be employed.

The dimodal crosslinkers that can be employed in the process of this invention are agents that have at least one polymerizable vinyl group and at least one functional group capable of reacting with carboxyl groups. To distinguish these from normal vinyl crosslinkers, we call them "dimodal crosslinkers," because they use two different modes of reaction to form a crosslink. Examples of dimodal crosslinkers include hydroxyethyl methacrylate, polyethylene glycol monomethacrylate, glycidyl methacrylate, and allyl glycidyl ether. Many examples of these type of agents are given in U.S. Patents 4,962,172 and 5,147,956 which teach the manufacture of absorbent films and fibers by (1) the preparation of linear copolymers of acrylic acid and hydroxyl containing monomers, (2) forming solutions of these copolymers into the desired shapes, and (3) fixing the shape by heating the polymer to form ester crosslinks between the pendant hydroxyl and carboxyl groups. In the current invention the dimodal crosslinkers advantageously are added homogeneously to the polymerization mixture at the start of the process. Preferred dimodal crosslinkers include hydroxyethyl (meth)acrylate, polyethylene glycol 400 monomethacrylate, glycidyl methacrylate. Hydroxyethyl (meth)acrylate is an example of a more preferred dimodal crosslinker. Mixture of dimodal crosslinkers can be employed.

Combinations of crosslinkers can be employed. The total amount of all crosslinkers present is sufficient to provide a polymer with good absorptive capacity, good absorption under load, and a low percent of extractable materials. Preferably the crosslinkers are present in an amount of 1,000 parts per million or more by weight based on the amount of the polymerizable monomer present, more preferably 2,000 ppm or more and most preferably 4,000 ppm or greater. Preferably, the crosslinkers are present in an amount of 50,000 parts per million or less by weight based upon the amount of the polymerizable monomer present, more preferably in amounts of 20,000 ppm or less and most preferably 15,000 ppm or less.

In those embodiments of the invention that utilize a blend of polyvinyl crosslinkers with non-vinyl and or dimodal crosslinkers, the effect on heat-treated capacity of all three types of crosslinkers is additive in nature. That is, if the amount of one crosslinker is increased the amount of another must be decreased to maintain the same overall heat-treated capacity. In addition, the proportion of the crosslinker components within the blend may be varied to achieve different polymer properties and processing characteristics. In particular the polyvinyl crosslinkers are typically more expensive than non-vinyl or dimodal crosslinkers. Therefore, the overall cost of the polymer is reduced if a greater proportion of the crosslinker blend is composed of less expensive non-vinyl and or dimodal crosslinkers. However, the non-vinyl and dimodal crosslinkers function essentially as latent crosslinkers. That is, the crosslinking imparted to the polymer by these agents is essentially not developed or seen until after a heat-treatment step. Little if any toughness is added to the hydrogel immediately after polymerization by use of such latent crosslinkers. This is an important concern for those processes for which a "tough" gel is desirable.

If too little of the total crosslinker blend is composed of polyvinyl crosslinker the polymerized hydrogel may not have sufficient toughness to be easily ground, processed, and dried. For this reason the proportion of polyvinyl crosslinker in the total crosslinker blend is preferably at least sufficient to produce a hydrogel that has enough toughness to be readily ground, processed, and dried. This toughness is inversely proportional to the centrifuged capacity of the polymer after drying but before heat-treatment. The exact amount of polyvinyl crosslinker required in the blend to achieve this level of toughness will vary, but is enough to provide a centrifuged absorption capacity of the polymer after drying but before heat-treatment of at least 10 g/g and preferably 45 g/g or less, more preferably 40 g/g or less, and most preferably 35 g/g or less.

Conventional additives which are well known in the art such as surfactants may be incorporated into the polymerization mixture. Polymerization can be accomplished under polymerization conditions in an aqueous or nonaqueous polymerization medium or in a mixed aqueous/nonaqueous polymerization medium. Polymerization accomplished by processes which employ nonaqueous polymerization media may use various inert hydrophobic liquids which are not miscible with water, such as hydrocarbons and substituted hydrocarbons including halogenated hydrocarbons as well as liquid hydrocarbons having from 4 to 20 carbon atoms per molecule including aromatic and aliphatic hydrocarbons, as well as mixtures of any of the aforementioned media.

In one embodiment, the polymer particles are prepared by contacting the monomers and crosslinkers of the invention in an aqueous medium in the presence of a free radical or oxidation reduction (redox) catalyst system and optionally a chlorine- or bromine-containing oxidizing agent under conditions such that a crosslinked hydrophilic polymer is prepared. As used herein, the term "aqueous medium" means water, or water in admixture with a water-miscible solvent. Such water-miscible solvents include lower alcohols and alkylene glycols. Preferably the aqueous medium is water.

The monomers and crosslinkers are preferably dissolved, dispersed or suspended in a suitable polymerization medium, such as, for example, the aqueous medium, at a concentration level of 15 percent by weight or greater, more preferably 25 percent or greater, and most preferably 29 percent or greater. The monomers and crosslinkers are preferably dissolved, dispersed or suspended in the aqueous medium.

Another component of the aqueous medium used to prepare the superabsorbent polymers comprises a free radical initiator, which may be any conventional water soluble polymerization initiator including, for example, peroxygen compounds such as sodium, potassium and ammonium peroxodisulfates, caprylyl peroxide, benzoyl peroxide, hydrogen peroxide, cumenc hydroperoxide, tertiary butyl diperphthalate, tertiary butyl perbenzoate, sodium peracetate and sodium percarbonate. Conventional redox initiator systems can also be utilized, which are formed by combining the foregoing peroxygen compounds with reducing agents, such as, for example, sodium bisulfite, sodium thiosulfate, L- or iso-ascorbic acid or a salt thereof or ferrous salts. The initiator can comprise up to 5 mole percent based on the total moles of polymerizable monomer present. More preferably the initiator comprises from 0.001 to 0.5 mole percent based on the total moles of polymerizable monomer in the aqueous medium. Mixtures of initiators can be employed.

In one embodiment of the invention, at least one chlorine- or bromine-containing oxidizing agent is added to the monomer mixture or to the wet hydrogel in order to reduce the amount of residual monomers in the final polymer. It is preferably added to the monomer mixture. Preferred oxidizing agents are bromates, chlorates and chlorites. Preferably a chlorate or bromate salt is added. The counterion of the bromate or chlorate salt can be any counterion which does not significantly interfere in the preparation of the polymers or their performance. Preferably, the counterions are alkaline earth metals ions or alkali metal ions. More preferred counterions are the alkali metals, with potassium and sodium being even more preferred. Chlorine-containing oxidizing agents are preferred. The oxidizing agent is present in sufficient amount such that after heat-treatment the residual monomer level is reduced and the desired balance of centrifuged absorption capacity and absorption under load (AUL) is achieved.

The chlorine- or bromine-containing oxidizing agent is present in a sufficient amount such that after heat-treatment the desired balance of polymer properties is achieved. If too much of the oxidizing agent is used, the ultimate properties of the polymers are degraded. If an insufficient amount is added, the above-described property improvements do not occur and the absorptive capacity will be low. Preferably, 10 ppm by weight or greater of a chlorine- or bromine-containing oxidizing agent based on the total weight of monomers (a), (b) and (c) is added, more preferably 50 ppm or greater and even more preferably 100 ppm or greater and most preferably 200 ppm or greater. Desirably, the amount of a chlorine- or bromine-containing oxidizing agent added is 2000 ppm or less by weight based on the monomers, more desirably 1000 ppm or less, preferably 800 ppm or less and most preferably 500 or less.

The process of the invention may be performed in a batch manner wherein all of the reaction materials are contacted and the reaction proceeds, or it may take place with the continuous addition of one or more of the components during the reaction period. The polymerization mixture in the polymerization medium is subjected to polymerization conditions which are sufficient to produce the water-absorbent polymers.

Preferably, the reaction is performed under an inert gas atmosphere, for example, under nitrogen or argon. The reaction may be performed at any temperature at which polymerization occurs, preferably 0°C or greater, more preferably 25°C or greater and most preferably 50°C or greater. The reaction is conducted for a time sufficient to result in the desired conversion of monomer to crosslinked hydrophilic polymer. Preferably, the conversion is 85 percent or greater, more preferably 95 percent or greater and most preferably 98 percent or greater. Advantageously, initiation of the reaction occurs at a temperature of at least 0°C.

It is also possible to prepare the polymer of the current invention with the addition of recycled "fines" to the polymerization mixture. See U.S. Patent 5,342,899. The amount of fines added to the polymerization mixture is preferably less than 12 weight percent based on the amount of monomer in the polymerization mixture, more preferably less than 10 weight percent, and most preferably less than 8 weight percent.

It is also possible to carry out the polymerization process using multiphase polymerization processing techniques such as inverse emulsion polymerization or inverse suspension polymerization procedures. In the inverse emulsion polymerization or inverse suspension polymerization procedures, the aqueous reaction mixture as hereinbefore described is suspended in the form of tiny droplets in a matrix of a water-immiscible, inert organic solvent such as cyclohexane. Polymerization occurs in the aqueous phase, and suspensions or emulsions of this aqueous phase in an organic solvent permit better control of the exothermic heat of polymerization and further provide the flexibility of adding one or more of the aqueous reaction mixture components in a controlled manner to the organic phase.

Inverse suspension polymerization procedures are described in greater detail in Obayashi et al., U.S. Patent 4,340,706; Flesher et. al. U.S. Patent 4,506,052; and Stanley et al. U.S. Patent 5,744,564. When inverse suspension polymerization or inverse emulsion polymerization techniques are employed, additional ingredients such as surfactants, emulsifiers and polymerization stabilizers may be added to the overall polymerization mixture. When any process employing organic solvent is utilized, it is important that the hydrogel-forming polymer material recovered from such processes be treated to remove substantially all of the excess organic solvent. Preferably, the hydrogel-forming polymers contain no more than 0.5 percent by weight of residual organic solvent.

During polymerization, the polymer of the invention generally absorbs all of the aqueous reaction medium to form a hydrogel. The polymer is removed from the reactor in the form of an aqueous hydrogel. The term "hydrogel" as used herein refers to water swollen superabsorbent polymer or polymer particles. In preferred embodiments, hydrogels coming out of the reactor comprise 15 to 50 percent by weight polymer, with the remainder comprising water. In a more preferred embodiment the hydrogel comprises 25 to 45 percent polymer. The hydrogel is preferably processed into a particulate shape during the polymerization reaction process in the reactor by the agitator to facilitate the removal of the hydrogel from the reactor. Preferred particle sizes of the hydrogel range from 0.001 to 25 cm, more preferably from 0.05 to 10 cm. In multiphase polymerization, the superabsorbent polymer hydrogel particles may be recovered from the reaction medium by azeotropic distillation and/or filtration followed by drying. If recovered by filtration, then some means of removing the solvents present in the hydrogel must be used. Such means are commonly known in the art.

The polymer of the invention is in the form of particles. Preferably, the polymer is substantially free of silver cations that are exchanged in a zeolite or bonded in a water-insoluble inorganic phosphate.

After removal from the reactor, the hydrogel polymer is subjected to comminution, such as, for example, by a convenient mechanical means of particle size reduction, such as grinding, chopping, cutting or extrusion. The size of the gel particles after particle size reduction should be such that homogeneous drying of the particles can occur. Preferred particle sizes of the hydrogel range from 0.5 to 3 mm. This particle size reduction can be performed by any means known in the art which gives the desired result. Preferably, the particle size reduction is performed by extruding the hydrogel.

The comminuted hydrogel polymer particles are subjected to drying conditions to remove the remaining polymerization medium and any dispersing liquid including the optional solvent and substantially all of the water. Desirably, the moisture content of the polymer after drying to remove the polymerization medium and any dispersing liquid including the optional solvent and substantially all of the water is between zero and 20 weight percent, preferably between 5 and 10 weight percent.

The temperature at which the drying takes place is a temperature high enough such that the polymerization medium and liquid including water and optional solvent is removed in a reasonable time period, yet not so high so as to cause degradation of the polymer particles, such as by breaking of the crosslink bonds in the polymer. Preferably, the drying temperature is 180°C or less. Desirably, the temperature during drying is 100°C or above, preferably 120°C or above and more preferably 150°C or above. The drying time should be sufficient to remove substantially all of the water and optional solvent. Preferably, a minimum time for drying is 10 minutes or greater, with 15 minutes or greater being preferred. Preferably, the drying time is 60 minutes or less, with 25 minutes or less being more preferred. In a preferred embodiment, drying is performed under conditions such that water, and optional solvent, volatilizing away from the absorbent polymer particles is removed. This can be achieved by the use of vacuum techniques or by passing inert gases or air over or through the layers of polymer particles. In a preferred embodiment, the drying occurs in dryers in which heated air is blown through or over layers of the polymer particles. Preferred dryers are fluidized beds or belt dryers. Alternatively a drum dryer may be used. Alternatively the water may be removed by azeotropic distillation. Such techniques are well known in the art.

During drying, the superabsorbent polymer particles may form agglomerates and may then be subjected to comminution, such as, for example, by mechanical means for breaking up the agglomerates. In a preferred embodiment, the superabsorbent polymer particles arc subjected to mechanical particle reduction means. Such means can include chopping, cutting and/or grinding. The object is to reduce the particle size of the polymer particles to a particle size acceptable in the ultimate end use. In a preferred embodiment, the polymer particles are chopped and then ground. The final particle size is preferably 2 mm or less, more preferably 0.8 mm or less. Preferably the particles have a size of 0.01 mm or greater, more preferably 0.05 mm or greater. Dried superabsorbent polymer particles of the present invention can be used as the basis polymer for further surface crosslinking treatment, for example, using polyvalent cations like aluminum ions and/or using one of the crosslinkers mentioned above by coating and subsequent heating at elevated temperatures.

In one embodiment of the invention, the polymer particles are subjected to a heat-treatment step after drying and optional particle size reduction. Heat-treatment of the polymer provides a beneficial increase in the absorption under load (AUL) of the superabsorbent polymer, particularly the AUL under higher pressures. Suitable devices for heat-treatment include, but are not limited to, rotating disc dryers, fluid bed dryers, infrared dryers, agitated trough dryers, paddle dryers, vortex dryers, and disc dryers. One of ordinary skill in the art would vary the time and temperature of heat-treatment as appropriate for the heat transfer properties of the particular equipment used.

The time period and temperature of the heat-treatment step are chosen such that the absorption properties of the polymer are improved as desired. The polymers are desirably heat-treated at a temperature of 170°C or above, more desirably 180°C or above, preferably at 200°C or above and most preferably at 220°C or above. Below 170°C no improvement in the absorption properties is seen. The temperature should not be so high as to cause the polymers to degrade. Preferably, the temperature is 250°C or below and more preferably 235°C or below. The polymers are heated to the desired heat-treatment temperature and preferably maintained at such temperature for 1 minute or more and more preferably 5 minutes or more and most preferably 10 minutes or more. Below 1 minute no improvement in properties is generally seen. If the heating time is too long it becomes uneconomical and there is a risk that the polymer may be damaged. Preferably polymer particles are maintained at the desired temperature for 60 minutes or less, preferably 40 minutes or less. Above 60 minutes no significant improvement in properties is noticed. The properties of the polymer particles can be adjusted and tailored by adjustment of the temperature and the time of the heating step.

After heat-treatment the polymer particles may be difficult to handle due to static electricity. It may be desirable to rehumidify the particles to reduce or eliminate the effect of the static electricity. Methods of humidification of dry polymers are well known in the art. In a preferred mode, the dry particles are contacted with water vapor. The dry particles are contacted with a sufficient amount of water to reduce or eliminate the effects of the static electricity, yet not so much so as to cause the particles to agglomerate. Preferably, the dry particles are humidified with 0.3 percent or more by weight of water and more preferably 5 percent or more by weight of water. Preferably, the dry particles are humidified with 10 percent or less by weight of water and more preferably 6 percent or less by weight of water. Optionally, agglomeration prevention or rehydration additives may be added to the crosslinked hydrophilic polymer. Such additives are well known in the art and include surfactants and inert inorganic particles such as silica; see, for example, U.S. Patents 4,286,082; 4,734,478; and DE 2706135. Remoisturization can also be accomplished using certain salt solutions as taught in EP 0 979 250.

According to the process of this invention for the preparation of superabsorbent polymers with odor control properties the silver salt is added to the process as a solution. Depending on whether water-soluble or water-insoluble silver salts or a mixture of both types are used they are dissolved in water, organic solvents or mixtures of both. Preferably, a soluble silver salt is added in the form of an aqueous solution. The silver salt is advantageously added in an amount providing 1 to 10,000 ppm silver in the final polymers. The concentration of the silver salt in the solution is not critical. Desirable concentrations of the silver salt in water range from 0.01 to 20 weight percent. The amount of silver solution preferably ranges from 0.1 to 10 weight percent, more preferably from 1 to 6 weight percent, based on dry polymer.

The silver salt may be added to the polymerization mixture (i) prior to the beginning of the polymerization or to the reaction mixture during polymerization, or (ii) to the crosslinked hydrogel prior to or after comminution, or (iii) to the dried polymer particles prior to or after heat-treatment, if a heat-treatment step is performed. It is also within the scope of the present invention to add the silver salt several times at various stages of the preparation process.

In one embodiment of the invention, the silver salt solution is added to the crosslinked wet hydrogel prior to or after comminution, and it is preferably sprayed onto the gel. Preferably, the silver ions are distributed substantially uniformly throughout the superabsorbent polymer particles rather than concentrated on the surfaces.

It is preferred to add the silver salt solution to the dried polymer particles which are optionally heat-treated. The silver ions are then distributed on and adsorbed to the polymer particle surfaces because their migration into the inner particle region is limited. Additional mixing means, for example, agitating and stirring, may be applied to improve the distribution of the silver ions on the surfaces of the polymer particles. The silver ions located on the polymer particle surfaces can be released when contacted with a liquid such as bacteria-infected urine, and this represents an economical use of odor controlling agents.

If an aqueous solution of the silver salt is added to the dried and optionally heat-treated polymer the solution may additionally contain a dust control agent, for example a propoxylated polyol as described in U.S. Patents 6,323,252 and 5,994,440. The propoxylated polyols are particularly suitable for binding the fine dust of the final superabsorbent polymer particles without causing agglomeration, and for binding the fine particles of powdery additives on the surface. The addition of the propoxylated polyol further results in a more homogeneous distribution of the silver salt solution or other aqueous additives on the surface of the superabsorbent polymer particles in the absence of organic solvent. Exemplary propoxylated polyols are available from The Dow Chemical Company under the brand name VORANOL. The propoxylated polyol is advantageously used in an amount of from 500 to 2,500 ppm, based on the weight of dry polymer. The concentration of the propoxylated polyol in water preferably ranges from 1 to 10 weight percent and more preferably from 3 to 6 weight percent.

In one embodiment the dried and optionally heat-treated polymer particles arc surface treated with aluminum sulfate. The aluminum sulfate may be added as an aqueous solution prior to or after the addition of the silver salt or the aluminum sulfate may be added to the aqueous silver salt solution and thus applied to the polymer together with the silver salt. The aluminum sulfate is preferably used in an amount of from 0.1 to 10 weight percent, based on dry polymer and its concentration in water is desirably from 5 to 49 weight percent. The use of an aqueous silver salt solution comprising both a propoxylated polyol and aluminum sulfate is especially preferred.

Other additives to which some odor control function is attributed may be used in addition to the silver salt. The additional additives may be added to the dried and optionally heat-treated polymers prior to, simultaneously with or after the addition of the silver salt solution. Exemplary additives are activated carbon, chlorophyllin, chelating agents, soda, sodium bicarbonate, copper sulfate, copper acetate, zinc sulfate, silicates, clay, cyclodextrin, citric acid, chitosan, ion exchange resin particles or combinations thereof. Zeolites may also be used in addition to the silver salt whereby the zeolite is not pretreated with the silver salt, that is, the zeolite is not ion exchanged with the silver cations.

To increase the flowability of the dried and optionally heat-treated polymer particles, silicon dioxide, preferably fumed silica, or other fine inorganic or organic powders may be mixed with the polymer particles. Powdery additives are desirably added to and mixed with the polymer particles together with the fumed silica. The fumed silica is preferably used in amounts of from 0.01 to 5 weight percent, and more preferably from 0.05 to 3 weight percent, all based on dry polymer. An exemplary fumed silica is Aerosil R972, available from Dcgussa AG, Germany. The additives may be added dry or in dispersed form, such as in the form of an aqueous dispersion.

In yet another embodiment dried and optionally heat-treated silver-free polymers are combined with silver-treated superabsorbent polymer. The silver-treated superabsorbent polymer can be normally-sized material or can be "fines" or mixture of these. "Fines" are superabsorbent polymer particles that are created from drying, grinding, and natural attrition during transport and heat-treating process of the typical gel process. The fine particle size fraction is in general undesirably small and therefore not suitable for incorporation in personal care article such as diapers, as described in U.S. Patent 5,342,899. This fine particle size fraction is often small enough to create dusting problems in production and a source of performance deterioration due to the well-known gel blocking tendency upon initial wetting. In a preferred embodiment, silver treated 'fines' are superabsorbent polymer particles which preferably pass through a 45 mesh (350 µm) screen and have been optionally heated to a temperature of from 170 to 250°C for from 1 to 60 minutes prior to the addition of a silver salt solution as described above.

The water-absorbent polymers of this invention can be used in any use wherein absorption and binding of aqueous fluids is desired and is especially suitable for such applications where it would be desirable to inhibit the development of malodor. In a preferred embodiment, the superabsorbent polymer particles of this invention are mixed into or attached to a structure of absorbent material such as synthetic or natural fibers or paper-based woven or nonwoven fibers to form a structure. In such a structure the woven or nonwoven structure functions as a mechanism for wicking and transporting fluid via capillary action to the superabsorbent polymer particles which bind and retain such fluids. Examples of such structures are sanitary napkins, diapers, and adult incontinence structures. In addition, there are various applications of the superabsorbent polymers with odor control property in non-personal care applications, for example, in medical care, agriculture, horticulture, gardening, pet litter, fertilizer, packaging and food packaging.

The absorbent structures according to the present invention comprise means to contain the superabsorbent polymer particles having odor control property. Any means capable of containing the described superabsorbent polymer particles, which means is further capable of being positioned in a device such as an absorbent garment, is suitable for use in the present invention. Many such containment means are known to those skilled in the art. For example, the containment means may comprise a fibrous matrix such as an airlaid or wetlaid web of cellulosic fibers, a meltblown web of synthetic polymeric fibers, a spunbonded web of synthetic polymeric fibers, a coformed matrix comprising cellulosic fibers and fibers formed from a synthetic polymeric material, airlaid heat-fused webs of synthetic polymeric material or open-celled foams. In one embodiment, it is preferred that the fibrous matrix comprise less than 10 preferably less than 5 weight percent of cellulosic fibers. Further, the containment means may comprise a support structure, such as a polymeric film, on which the superabsorbent polymer particles is affixed. The superabsorbent polymer particles may be affixed to one or both sides of the support structure which may be water-pervious or water-impervious.

The absorbent structures according to the present invention are suited to absorb many fluids including body fluids such as, for example, urine, menses, and blood and are suited for use in absorbent garments such as diapers, adult incontinent products and bed pads; in catamenial devices such as sanitary napkins and tampons; and in other absorbent products such as, for example, wipes, bibs and wound dressings. Accordingly, in another aspect, the present invention relates to an absorbent garment comprising an absorbent structure as described above.

The following examples are included to illustrate the invention, and do not limit the scope of the Claims. All parts and percentages are by weight unless otherwise stated.

### Test Methods

### Method for Microbiological Evaluation

### Preparation of Bacterial Strain Suspensions

The following analytical grade components were added to a vessel and stirred for 15 minutes to make 10 kg of synthetic urine solution:
200 g urea
90 g NaCl
11.0 g Mg₂SO₄·7H₂O
7.95 g CaCl₂·2H₂O
9691.0 g distilled water

A synthetic urine medium that simulates real urine in which various bacteria strains can proliferate was developed for use in these experiments. Peptone (tryptone soya broth (Oxoid Company, UK)) was found to be useful as a nutrition medium for bacteria proliferation in the synthetic urine solution.

A peptone solution was prepared by dissolving 60 g of tryptone soya broth powder (product code: CM 129, Oxoid company, UK) in 1000 g distilled water with thorough mixing. The solution was then sterilized by autoclaving at 121ºC for 20 minutes.

A culture medium was prepared by adding 4 g of the peptone solution to 400 g of synthetic urine solution in a 1000 ml Erlenmeyer flask prior to the start of culture growth; this corresponds to a peptone concentration of 0.60 g in 1000 g synthetic urine. The culture medium was inoculated with 2-3 bacterial colonies from Columbia sheep blood (5 percent) agar plates (Becton Dickinson) which had been kept at 4°C not longer than 1 week. In the case of Proteus mirabilis an approximately equivalent amount of bacteria was used.

To start culture growth, each flask containing an inoculated culture medium at 4°C was placed into an incubator at 38°C. It took about 14 hours for the temperature of the inoculated culture medium to rise to 38°C.

The following bacteria strains were employed: Escherichia coli (hereinafter EC), an ATTC 25922 (American Tissue Type Culture Collection) type strain; Proteus mirabilis (hereinafter PM) (ATTC 14153); and Klebsiella pneumoniae (hereinafter KP) (ATTC 10031). For the tests conducted below, each cultured strain was used either as a single bacteria strain suspension, or was mixed with suspensions of the other 2 strains to make a mixture having an equal volume of each of the three suspensions. The mixture of suspensions had a total bacterial content approximately equal to the total bacterial content of a single strain suspension.

The CFU (colony forming unit) of the cultures was then determined by viable plate counts.

### CFU (Colony Forming Unit) Analysis

Polymer samples having a particle size fraction between 100 to 800 µm were used for CFU analysis unless otherwise stated. The CFU count was determined using the following procedure. 5.00 g of each polymer sample were placed into a 500 ml glass bottle containing 150 ml of the single or the mixed bacteria strain suspension (PM, EC, KP). The mixture of polymer and bacteria suspension was then stirred (100 rpm) using a dumb-bell shaped magnetic stirrer having length of 5 cm until the stirring was stopped by swelling polymer gels (time zero). 1 g of the swollen gel was taken and was placed into a small plastic tube with a screw cap. 10 ml of 0.9 percent NaCl solution were added to the tube, followed by immediate, vigorous shaking of the tube. Using 0.9 percent sodium chloride solution, the supernatant was then used for a further series of dilution, for example, the final dilution of 1,000 and 10,000 fold, wherein the dilution with 10 ml of 0.9 percent sodium chloride solution above was included. Unless otherwise stated, the CFU results were those obtained from the 10,000 fold dilution. For better comparison, in some cases the result of the 1,000 fold dilution was restandardized to that for the 10,000 fold dilution which results in CFU numbers being smaller than 1. 25 µl of each diluted solution were put onto a plate, and CFUs were counted after incubation for 24 h at 38°C. The CFU analysis was performed 0, 4 and 24 h after time zero. In all experiments, the samples of the present invention were analyzed for CFU together with the pure bacteria suspension and a control polymer sample. The CFU analysis was performed in duplicate, and the arithmetic mean was taken in all cases.

### Ammonia Test using DRAEGER Tube

150 ml of bacteria suspension were placed in a 500 ml glass bottle and a 5.5 cm bone-shaped magnetic stirring bar was added. 5 g of polymer was put into a beaker. The bottle was placed on the stirrer (100 rpm). The polymer was added to the bottle which was then closed with a three-opening screw cap. When the stirring action was stopped by swelling polymer gels (time zero), the bottle was then placed in a lab oven that was prewarmed at 38°C. First a 3 cm length of silicon tubing, then a DRAEGER (Trademark of Draeger Company, Germany) test tube for the ammonia test were attached to one opening of the bottle. The ammonia concentration in the head space was indicated as a color change on a scale graded in ppm, and was read as a function of time at 38°C.

### Sniff Test

The sniff test was done by a single, experienced laboratory technician. Bacteria-inoculated gel samples were prepared by mixing 5 g polymer with 150 ml of the inoculated culture medium prepared above. The sniff test was performed using bacteria-inoculated gel samples after incubation of the polymer for 24 hours at 38°C. The following ratings were used for describing the odor of the polymer gels.

**Table 1: Degrees of Sniff Test Results**

| Degree | Odor Description |
|---|---|
| +++ | Very Strong ammonia odor, very strongly malodorous |
| ++ | Strong ammonia odor, strongly malodorous |
| + | Ammonia odor, malodorous |
| 0 | Non ammonia odor, non malodorous |

### Polymers

Polymer A is DRYTECH S230R brand superabsorbent polymer which is commercially available from Dow Deutschland GmbH & Co. OHG. It had a degree of neutralization of about 68 mol percent. It had a particle size fraction between 100 and 800 µm.

Polymer B is a non-heat-treated superabsorbent polymer DRYTECH XZS 91041.00 prepared by Dow Deutschland GmbH & Co. OHG. It was prepared by a batch process of gel polymerization in accordance with steps (I) to (III) of the present invention and had a degree of neutralization of about 68 mol percent. It had a particle size fraction between 100 and 800 µm.

### Sample Preparation Procedure

The following procedure was employed for all experiments except as otherwise noted.

Dry Polymer A powder (1.2 kg) was placed at room temperature into a 5 liter laboratory scale blender (Loedige Company, Germany). Fumed silica (3.0g) (AEROSIL R972, available from Degussa-Huels Company, Germany) was added to the polymer powder to increase flowability. When other powder additives, for example, cyclodextrin, activated carbon, chlorophyllin, etc. were used, they were added to the mixture of the polymer and fumed silica. The blender contents were then blended for 15 minutes.

The required amount of water-soluble salt or other water-soluble additive, was dissolved in a mixture of 36 g of deionized water and 1.14 g of VORANOL CP 755 brand propoxylated polyol (VORANOL is a trademark of The Dow Chemical Company). The resulting aqueous fluid was then sprayed directly into the Loedige blender during agitation (126 rpm) and the whole mixture was blended for a further 15 minutes before unloading. The CFU (Colony Forming Unit) Analysis, as described hereinabove, was then performed using the mixed bacteria strain suspension.

The silver ion concentrations in all following tables were based on dry polymer. In all of the following experiments the "control" sample was the corresponding inoculated polymer without an odor control additive. Experiments having the designation "-0" represent the bacterial suspension with neither polymer nor additives. All examples marked as * are comparative experiments and are not examples of the present invention.

### Experiment Series 1

The Sample Preparation Procedure was followed using 0.189 g silver nitrate salt (100 ppm of silver ions, based on dry polymer) (Ex 1-1). In Example 1-2, 24 g of β-cyclodextrin (2 percent, based on dry polymer) was employed as a dry additive as a comparative experiment. The bacteria suspension used was a single Proteus mirabilis (PM) strain. The ammonia concentrations in the head space of the various samples were measured using the "Ammonia Test using Draeger Tube", and the results are summarized in Table 2.

**Table 2: Cyclodextrin and Silver Ion Treatment -Ammonia Concentration (ppm) as a Function of Time using Single Proteus Mirabilis (PM) Strain Suspension**

| | NH₃ | | | |
|---|---|---|---|---|
| Time (h) | Ex 1-0 Bac. Suspension (PM) | Control 1* | Ex 1-1 (100 ppm Ag⁺⁾ | Ex 1-2* (2 percent β-cyclodextrin) |
| 0 | 0 | 0 | 0 | 0 |
| 0.83 | 0 | 0 | 0 | 0 |
| 1.5 | 1.5 | 0 | 0 | 0 |
| 2.5 | 2 | 0 | 0 | 0 |
| 3.5 | 2.5 | 0 | 0 | 0 |
| 4.5 | 3.3 | 0 | 0 | 0 |
| 5.17 | 4.3 | 0 | 0 | 0 |
| 18.67 | 65 | 4.8 | 0 | 5.3 |
| 19.5 | 70 | 5 | 0 | 7.5 |
| 20.17 | 73 | 6.3 | 0 | 9 |
| 21 | 83 | 9 | 0 | 16 |
| 22 | - | 12 | 0 | 20 |
| 23 | - | 15 | 0 | 24 |
| 24 | - | 18 | 0 | 29 |
| 25.08 | - | 20 | 0 | 32 |
| 26.17 | - | 25 | 0 | 39 |
| 27 | - | 27 | 0 | 42.5 |
| 27.42 | - | 28 | 0 | 45 |
| 41.3 | - | 71.7 | 0 | 115 |
| 45.47 | - | 97.5 | 0 | - |
| 47.55 | - | 105 | - | - |

The material of Ex 1-1 of the present invention showed no detectable ammonia concentration in the head space over a prolonged time period. This material was superior to the untreated control sample and the sample comprising 2 percent β-cyclodextrin (Ex 1-2). Interestingly, the cyclodextrin-containing polymer showed an even higher ammonia concentration than the control sample. This fact is partly explained by the fact that cyclodextrin can be easily metabolized by the bacteria employed.

### Experiment Series 2

The Sample Preparation Procedure was followed using varying amounts of silver nitrate.

**Table 3: Addition of Various Amounts of Silver Nitrate - CFU Counts for Various Polymers comprising Different Silver Ion Concentrations (Bacteria Strain Mixture of PM, EC, KP)**

| Sample | Time | CFU |
|---|---|---|
| Ex 2-0 Bacteria Strain Suspension (Mixture of PM, EC and KP)* | 0 h | 21.5 |
| | 4 h | 24 |
| | 24 h | 23.5 |
| Control 2* | 0 h | 21 |
| | 4 h | 24.5 |
| | 24 h | 61 |
| Ex 2-1 25 ppm Ag⁺= 0.00472 g AgNO₃ | 0 h | 23 |
| | 4 h | 2 |
| | 24 h | 0.3 |
| Ex 2-2 50 ppm Ag⁺= 0.0945 g AgNO₃ | 0 h | 29 |
| | 4 h | 1.1 |
| | 24 h | 0 |
| Ex 2-3 100 ppm Ag⁺= 0.1890 g AgNO₃ | 0 h | 25 |
| | 4 h | 0.1 |
| | 24 h | 0 |
| Ex 2-4 250 ppm Ag⁺= 0.4724 g AgNO₃ | 0 h | 26.5 |
| | 4 h | 0 |
| | 24 h | 0 |
| Ex 2-5 500 ppm Ag⁺= 0.9449 g AgNO₃ | 0 h | 32 |
| | 4 h | 0.05 |
| | 24 h | 0 |
| Ex 2-6 1,000 ppm Ag⁺= 1.8898 g AgNO₃ | 0 h | 10 |
| | 4 h | 0 |
| | 24 h | 0 |

In many of the following experiments, Ex 2-3 was repeated as a reference point for clarifying the antibacterial effects of the different treatments.

### Experiment Series 3

**Table 4: CFU Counts for Various Polymers using a Mixture of Bacteria Strains Suspension (PM, EC, KP)**

| Sample | Time | CFU | Odor |
|---|---|---|---|
| Ex 3-0 Bacteria Strains Suspension (PM, EC, KP)* | 0 h | 191 | +++ |
| | 4 h | 154.5 | |
| | 24 h | 38.5 | |
| Control 3* | 0 h | 151.5 | ++ |
| | 4 h | 208.5 | |
| | 24 h | 254 | |
| Ex 3-1* no Ag⁺ ions, only fumed silica as additive | 0 h | 117.5 | ++ |
| | 4 h | 212 | |
| | 24 h | 253 | |
| Ex 3-2 100 ppm Ag⁺ = 0.1890 g AgNO₃ | 0 h | 176 | 0 |
| | 4 h | 0.85 | |
| | 24 h | 0 | |

Comparing the control sample with Ex 3-1 indicates that the fumed silica had no effect on the CFU or malodor. Thus, the odor control property shown in Ex 3-2 has to be attributed to the presence of silver ions.

### Experiment Series 4

The Sample Preparation Procedure was followed using CuSO₄·5H₂O or Cu(O₂CCH₃)₂·H₂O (copper acetate).

**Table 5: Silver Ion and Copper Ion Treatment - CFU Counts for Various Polymers using a Mixture of the Bacteria Strains Suspension (PM, EC and KP)**

| Sample | Time | CFU | Odor |
|---|---|---|---|
| Ex 4-0 Bacteria Strain Suspension (Mixture of PM, EC and KP)* | 0h | 73 | +++ |
| | 4h | 94 | |
| | 24h | 33 | |
| Control 4* | 0h | 70 | ++ |
| | 4h | 264 | |
| | 24h | 211 | |
| Ex 4-1* 0.25 percent Cu²⁺ = 12 g CuSO₄·5H₂O | 0h | 104 | + |
| | 4h | 195 | |
| | 24h | 244 | |
| Ex 4-2* 0.06 percent Cu²⁺= 2.4 g Cu(O₂CCH₃)₂·H₂O | 0h | 98 | ++ |
| | 4h | 184 | |
| | 24h | 121 | |
| Ex 4-3 100 ppm Ag⁺ = 0.1890 g AgNO₃ | 0h | 57 | 0 |
| | 4h | 0 | |
| | 24h | 0 | |

The results show that treatment with the copper ions of copper sulfate and copper acetate was not as effective at reducing the CFU or the odor, compared to treatment with silver ions.

### Experiment Series 5

The Sample Preparation Procedure was followed using varying amounts of β-cyclodextrin.

**Table 6: Cyclodextrin and Silver Ion Treatment - CFU Counts for Various Polymers using a Mixture of Bacteria Strains Suspension (PM, EC, KP)**

| Sample | Time | CFU | Odor |
|---|---|---|---|
| Ex 5-0 Bacteria Strain Suspension (Mixture of PM, EC and KP)* | 0h | 61 | +++ |
| | 4h | 68 | |
| | 24h | 75 | |
| Control 5* | 0h | 51 | ++ |
| | 4h | 129 | |
| | 24h | 293.5 | |
| Ex 5-1* 1 percent β-Cyclodextrin = 12 g | 0h | 45.5 | + |
| | 4h | 146 | |
| | 24h | 252.5 | |
| Ex5-2* 2 percent β-Cyclodextrin = 24 g | 0h | 24.5 | ++ |
| | 4h | 141.5 | |
| | 24h | 309.5 | |
| Ex 5-3 2 percent β-Cyclodextrin + 100 ppm Ag⁺ | 0h | 24 | 0 |
| | 4h | 0.25 | |
| | 24h | 0 | |
| Ex 5-4 100 ppm Ag⁺ = 0.1890 g AgNO₃ | 0h | 32.5 | 0 |
| | 4h | 4 | |
| | 24h | 0 | |

β-Cyclodextrin reduced neither CFU nor odor, but seemed to have a negative impact on the CFU and odor when used as a simple additive to the polymer. The combined use of β-cyclodextrin and silver ions provided a product with odor control properties.

### Experiment Series 6

The Sample Preparation Procedure was followed using activated carbon or chlorophyllin powder.

**Table 7: Polymers Treated with Activated Carbon or Chlorophyllin-CFU Counts for Various Polymers using a Mixture of bacteria Strains Suspension (PM, EC, KP)**

| Sample | Time | CFU | Odor |
|---|---|---|---|
| Ex 6-0 Bacteria Strain Suspension (Mixture of PM, EC and KP)* | 0h | 42.5 | +++ |
| | 4h | 75.5 | |
| | 24h | 29 | |
| Control 6* | 0h | 41 | ++ |
| | 4h | 138.5 | |
| | 24h | 295 | |
| Ex 6-1 * 1 percent activated carbon = 12 g | 0h | 48 | + |
| | 4h | 228.5 | |
| | 24h | 316.5 | |
| Ex 6-2* 1 percent chlorophyllin = 12 g | 0h | 53.5 | + |
| | 4h | 122.5 | |
| | 24h | 518 | |
| Ex 6-3 100 ppm Ag⁺ = 0.1890 g AgNO₃ | 0h | 39.5 | 0 |
| | 4h | 0.2 | |
| | 24h | 0 | |

The treatment with activated carbon and chlorophyllin showed a slight reduced odor but resulted in an increased CFU which indicates the high proliferation of bacteria.

### Experiment Series 7

The Sample Preparation Procedure was followed using varying amounts of the chelating agent VERSENEX 80 (The Dow Chemical Company, 40.2 percent aqueous solution of the pentasodium salt of diethylene triamine pentaacetic acid).

**Table 8: Polymers Treated with Silver Ions and VERSENEX 80 - CFU Counts for Various Polymers using a Mixture of Bacteria Strains Suspension (PM, EC, KP)**

| Sample | Time | CFU | Odor |
|---|---|---|---|
| Ex 7-0 Bacteria Strain Suspension (Mixture of PM, EC and KP)* | 0h | 70 | +++ |
| | 4h | 78 | |
| | 24h | 57.5 | |
| Control 7* | 0h | 101.5 | ++ |
| | 4h | 129.5 | |
| | 24h | 186.5 | |
| Ex 7-1 * 1250 ppm VERSENEX 80 = 3.75 g of solution | 0h | 80 | + |
| | 4h | 158.5 | |
| | 24h | 215 | |
| Ex 7-2* 2500 ppm VERSENEX 80 = 7.5 g of solution | 0h | 104.5 | + |
| | 4h | 136 | |
| | 24h | 239 | |
| Ex 7-3* 5000 ppm VERSENEX 80 = 15.0 g of solution | 0h | 64.5 | + |
| | 4h | 142 | |
| | 24h | 135.5 | |
| Ex 7-4* 7500 ppm VERSENEX 80 = 22.4 g of solution | 0h | 93.5 | ++ |
| | 4h | 167.5 | |
| | 24h | 208 | |
| Ex 7-5* 10000 ppm VERSENEX 80 = 29.85 g of solution | 0h | 97 | ++ |
| | 4h | 137.5 | |
| | 24h | 195.5 | |
| Ex 7-6 75 ppm Ag⁺ = 0.1415 g AgNO₃ | 0h | 87.5 | 0 |
| | 4h | 2 | |
| | 24h | 0 | |
| Ex 7-7 5000 ppm VERSENEX 80 + 75 ppm Ag⁺ | 0h | 114.5 | 0 |
| | 4h | 2.5 | |
| | 24h | 0 | |
| Ex 7-8 100 ppm Ag⁺ = 0.1890 g AgNO₃ | 0h | 66.5 | 0 |
| | 4h | 07 | |
| | 24h | 0 | |

No effect of the chelating agent VERSENEX 80 was observed within the concentration range between 0 and 10,000 ppm when it was used as the sole additive to the polymer. The combined use of VERSENEX 80 and silver ion resulted in a polymer with odor control properties.

### Experiment Series 8

The Sample Preparation Procedure was followed, except as noted, using Na₂CO₃ or NaHCO₃ powder. In Ex 8-1 glycine was added to the solution of the polyol. In Ex 8-2 and 8-3 only 12 g (1 percent) of fumed silica was used.

**Table 9: Glycine, Na₂CO₃ and NaHCO₃ Treated Polymers - CFU Counts for Various Polymers using a Mixture of Bacteria Strains Suspension (PM, EC, KP)**

| Sample | Time | CFU | Odor |
|---|---|---|---|
| Ex 8-0 Bacteria Strain Suspension (Mixture of PM, EC and KP)* | 0h | 52.5 | +++ |
| | 4h | 57 | |
| | 24h | 52.5 | |
| Control 8* | 0h | 49.5 | ++ |
| | 4h | 205 | |
| | 24h | 266 | |
| Ex 8-1 * 1 percent glycine = 12 g | 0h | 54.5 | + |
| | 4h | 188 | |
| | 24h | 169 | |
| Ex 8-2* 5 percent Na₂CO₃ = 60 g | 0h | 47 | + |
| | 4h | 165.5 | |
| | 24h | 219 | |
| Ex 8-3* 5 percent NaHCO₃ = 60 g | 0h | 40 | ++ |
| | 4h | 154 | |
| | 24h | 215 | |
| Ex 8-4 100 ppm Ag⁺ = 0.1890 g AgNO₃ | 0h | 24.5 | 0 |
| | 4h | 0 | |
| | 24h | 0 | |

### Experiment Series 9 and 10

The Sample Preparation Procedure was followed except that superabsorbent polymer samples with a neutralization degree of 35 percent for Series 9 and 50 percent for Series 10 were used. The polymers were prepared by a batch process using a polymerization reactor (LIST AG, Switzerland) with a stainless steel agitator assembly. The assembly allowed grinding of the gel formed during polymerization. The reactor was jacketed to allow for heating or cooling via a water-circulating heater (GWK, Germany). The reactor was equipped with a reflux condenser, a metal funnel, a nitrogen inlet tube, thermocouples and a vacuum pump. The gel mass in the reactor was cooled by pulling a vacuum. In all cases, polymer solids are kept at 35 percent. 180 kg of monomer mix were prepared using the materials listed in Table 10.

**Table 10: Polymerization Recipe for the Production of Superabsorbent Polymer Particles with a Neutralization Degree (ND) of 50 percent and 35 percent**

| Degree of Neutralization (ND) | | ND of 50 percent (Ex. Series 10) | ND of 35 percent (Ex. Series 9) |
|---|---|---|---|
| Component | Wt. percent of Component in Added Medium | Weight | Weight |
| Acrylic Acid | 99 | 55.2 kg | 57.5 kg |
| Sodium hydroxide | 50 | 30.7 kg | 22.4 kg |
| Water | | 91.9 kg | 98.7 kg |
| HE-TMPTA ⁽¹⁾ | 100 | 165 g | 172 g |
| VERSENEX 80 ⁽²⁾ | 40.2 | 68 g | 72 g |
| PEG-600-60 ⁽³⁾ | 100 | 165 g | 172 g |
| Hydrogen peroxide | 30 | 64 g | 67 g |
| Sodium Chlorate | 10 | 146 g | 152 g |
| Sodium peroxodisulfate | 10 | 938 g | 977 g |
| Ascorbic acid | 1 | 828 g | 862 g |

| | | | |
|---|---|---|---|
| (1) highly ethoxylated trimethylolpropane triacrylate (2) pentasodium salt of diethylene triamine pentaacetic acid (3) polyethylene glycol with an average molecular weight of 600 g/mol (available from Clariant International Ltd., Switzerland) | | | |

### Preparation of Monomer Mix

The polymerization procedure is described for the polymer having a neutralization degree of 50 percent. The same procedure was used for the polymer having a neutralization degree of 35 percent using the corresponding amounts indicated in Table 10. 27.6 kg of acrylic acid were added to 30.7 kg of the 50 wt percent sodium hydroxide solution and 77 kg of process water in such a way to prevent the temperature from exceeding 38ºC. 67 g of VERSENEX 80 were added to the pre-neutralized monomer mix. 166 g ofHE-TMPTA and 166 g of PEG-600 were dissolved in 27.6 kg of acrylic acid and poured into the pre-neutralized monomer mix after it was cooled to room temperature. 146 g of 10 wt percent aqueous sodium chlorate solution were added to the monomer mix. The resulting monomer mix was then pumped into the reactor and the reactor was evacuated once and purged with nitrogen.

The reactor charged with monomer mix was controlled at 25°C. The polymerizations were initiated in most cases at 25 ± 2°C by pouring 64 g of 30 wt percent aqueous hydrogen peroxide solution, 938 g of 10 wt percent sodium peroxodisulfatc aqueous solution and finally 828 g of 1 wt percent aqueous ascorbic acid solution into the reactor. Two to three minutes of mixing (agitation speed 20 rpm) were allowed between the addition of peroxodisulfate and ascorbic acid solutions and the agitation speed was reduced again to 10 rpm. The metal funnel was flushed after each addition with 500 ml of water. The reactor was evacuated/purged again before addition of ascorbic acid. A very slight positive nitrogen pressure was maintained in the reactor during polymerization in order to prevent oxygen from entering the reactor.

After addition of the ascorbic acid solution, the heating equipment was switched on and adjusted to 75°C. The temperature of the reaction mixture rose to a peak temperature of approximately 75°C over a period of about 20 minutes. After reaching the peak temperature, the gel was kept at 70°C for a further 60 minutes in the reactor under agitation.

The gel from the polymerizations was broken into small pieces using a laboratory extruder (MADO GmbH, Germany) and was dried in an air-forced air laboratory oven (HERAEUS) at 170°C for 2 h. The dried superabsorbent polymer was then ground using a Baumeister grinder (Baumeister GmbH, Germany) and sifted over 0.8 and 0.1 mm sieves.

### Heat-Treatment Procedure

Heat treatment was performed using a fluidized bed (Allgaier GmbH, Germany). Once the target temperature was reached and stabilized, approximately 1.8 kg of polymer sample were placed in the zone and a contact thermometer was placed in the sample. The temperature of the sample was monitored until it stabilized at the target temperature. The sample was maintained at the target temperature for the desired time.

In Ex 9-1 to 9-3 and the corresponding control sample, the polymer particles were heat-treated at 190°C for 30 minutes. The Sample Preparation Procedure was followed individually using AgNO₃, CuSO₄·5H₂O, and ZnSO₄·7H₂O.

**Table 11: 35 percent Neutralization Polymers Treated with Ag, Cu or Zn - CFU Counts for Various Polymers using a Mixture of Bacteria Strains Suspension (PM, EC, KP)**

| Sample | Time | CFU | Odor |
|---|---|---|---|
| Ex 9-0 Bacteria Strain Suspension (Mixture of PM, EC and KP)* | 0h | 52 | +++ |
| | 4h | 115 | |
| | 24h | 41.5 | |
| Control 9* | 0h | 91 | ++ |
| | 4h | 83 | |
| | 24h | 113.5 | |
| Ex 9-1 100 ppm Ag⁺ = 0.1890 g AgNO₃ | 0h | 76 | 0 |
| | 4h | 0.25 | |
| | 24h | 0.35 | |
| Ex 9-2* 0.25 percent Cu²⁺ = 12 g CuSO₄·5H₂O | 0h | 51 | + |
| | 4h | 20 | |
| | 24h | 38 | |
| Ex 9-3* 0.23 percent Zn²⁺ = 12 g ZnSO₄·7H₂O | 0h | 82 | + |
| | 4h | 58.5 | |
| | 24h | 145.5 | |

In Ex 10-1 to 10-3 and the corresponding control sample the polymer particles were heat-treated at 200°C for 30 minutes.

**Table 12: 50 percent Neutralization Polymers Treated with Ag, Cu or Zn - CFU Counts for Various Polymers using a Mixture of Bacteria Strains Suspension (PM, EC, KP)**

| Sample | Time | CFU | Odor |
|---|---|---|---|
| Ex 10-0 Bacteria Strain Suspension (Mixture of PM, EC and KP)* | 0h | 60.5 | +++ |
| | 4h | 101.5 | |
| | 24h | 52.5 | |
| Control 10* | 0h | 98 | ++ |
| | 4h | 175 | |
| | 24h | 183 | |
| Ex 10-1 100 ppm Ag⁺ = 0.1890 g AgNO₃ | 0h | 43.5 | 0 |
| | 4h | 3 | |
| | 24h | 0 | |
| Ex 10-2* 0.25 percent Cu²⁺ = 12 g CuSO₄ ·5H₂O | 0h | 41.5 | + |
| | 4h | 147.5 | |
| | 24h | 140 | |
| Ex 10-3* 0.23 percent Zn²⁺ = 12 g ZnSO₄·7H₂O | 0h | 56.5 | + |
| | 4h | 238.5 | |
| | 24h | 226.5 | |

It is evident from the results in Tables 11 and 12 that silver ions are much more effective for odor control than copper or zinc ions, regardless of the degree of neutralization.

### Experiment Series 11

Dry Polymer B, which is a non-heat-treated product, was surface treated using aluminum ions, and the treated polymer was then subsequently subjected to a further treatment with silver ions or other metal ions.

A 48.5 wt percent solution was prepared by dissolving 485 g Al₂(SO₄)₃·14H₂O in 515 g of distilled water. The Sample Preparation Procedure was followed except that no fumed silica was used when aluminum ion surface treatment was done. To each of the polymer samples, 75 g of the aluminum sulfate solution (6.25 percent, based on dry polymer) was sprayed directly onto the polymer and the mixture was blended for 15 minutes. The results are shown in Table 13.

**Table 13: Surface-Treated Polymers Treated with Ag, Cu or Zn - CFU Counts for Various Polymers using a Mixture of Bacteria Strains Suspension (PM, EC, KP)**

| Sample | Time | CFU | Odor |
|---|---|---|---|
| Ex 11-0 Bacteria Strain Suspension (Mixture of PM, EC and KP)* | 0h | 60.5 | +++ |
| | 4h | 101.5 | |
| | 24h | 52.5 | |
| Control 11* (Surface treatment with Al³⁺ ions) | 0h | 36 | ++ |
| | 4h | 81.5 | |
| | 24h | 43 | |
| Ex 11-1 100 ppm Ag⁺ = 0.1890 g AgNO₃ | 0h | 33 | 0 |
| | 4h | 0 | |
| | 24h | 0 | |
| Ex 11-2* 0.25 percent Cu²⁺ = 12 g CuSO₄ ·5H₂O | 0h | 39.5 | ++ |
| | 4h | 60.5 | |
| | 24h | 52 | |
| Ex 11-3* 0.23 percent Zn²⁺ = 12 g ZnSO₄·7H₂O | 0h | 40 | ++ |
| | 4h | 79.5 | |
| | 24h | 122.5 | |

The polymer surface-treated with aluminum ions was not effective for odor control. The addition of copper or zinc ions did not improve the odor control function while the addition of silver ion clearly showed a positive effect.

### Experiment Series 12, 13 and 14

The Sample Preparation Procedure was followed using various silver salts and silver colloid (Merck). The results are shown in Tables 14, 15 and 16, respectively.

**Table 14: Addition of Various Silver Salts (Ex. Series 12) - CFU Counts for Various Polymers using a Mixture of Bacteria Strains Suspension (PM, EC, KP)**

| Sample | Time | CFU | Odor |
|---|---|---|---|
| Ex 12-0 Bacteria Strain Suspension (Mixture of PM, EC and KP)* | 0h | 41.5 | +++ |
| | 4h | 126 | |
| | 24h | 89 | |
| Control 12* | 0h | 52 | ++ |
| | 4h | 226 | |
| | 24h | 106.5 | |
| Ex 12-1 0.186 g Ag acetate | 0h | 50 | 0 |
| | 4h | 4.5 | |
| | 24h | 0 | |
| Ex 12-2 0.255Ag benzoate | 0h | 73.5 | 0 |
| | 4h | 0.25 | |
| | 24h | 0.15 | |
| Ex 12-3 0.1890 g AgNO₃ | 0h | 74.5 | 0 |
| | 4h | 30.5 | |
| | 24h | 0 | |

**Table 15: Addition of Silver Sulfate and Silver Colloid (Ex. Series 13) - CFU Counts for Various Polymers using a Mixture of Bacteria Strains Suspension (PM, EC, KP)**

| Sample | Time | CFU | Odor |
|---|---|---|---|
| Ex 13-0 Bacteria Strain Suspension (Mixture of PM, EC and KP)* | 0h | 80.5 | +++ |
| | 4h | 101 | |
| | 24h | 51.5 | |
| Control 13* | 0h | 49 | ++ |
| | 4h | 126 | |
| | 24h | 176 | |
| Ex 13-1 0.347 g Ag₂SO₄ | 0h | 88 | 0 |
| | 4h | 0.2 | |
| | 24h | 0.6 | |
| Ex 13-2* 0.12 g Ag colloid | 0h | 81 | + |
| | 4h | 23 | |
| | 24h | 25.5 | |
| Ex 13-3 0.1890 g AgNO₃ | 0h | 79.5 | 0 |
| | 4h | 0.35 | |
| | 24h | 0 | |

**Table 16: Addition of Silver Sulfadiazin (Ex. Series 14) - CFU Counts for Various Polymers using a Mixture of Bacteria Strains Suspension (PM, EC, KP)**

| Sample | Time | CFU | Odor |
|---|---|---|---|
| Ex 14-0 Bacteria Strain Suspension (Mixture of PM, EC and KP)* | 0h | 41.5 | +++ |
| | 4h | 126 | |
| | 24h | 89 | |
| Control 14* | 0h | 52 | ++ |
| | 4h | 226 | |
| | 24h | 106.5 | |
| Ex 14-1 0.397 g Ag sulfadiazine | 0h | 50 | 0 |
| | 4h | 4.5 | |
| | 24h | 0 | |
| Ex 14-2 0.1890 g AgNO₃ | 0h | 73.5 | 0 |
| | 4h | 0.25 | |
| | 24h | 0.15 | |

The above CFU counts demonstrate that all tested aqueous solutions of silver salts were effective whereas the silver atoms in silver colloid had only a very small odor control effect.

### Experiment Series 15

In Ex 15-1 to 15-4 the effect of ALPHASAN RC 5000, which is a powdery water-insoluble silver-containing inorganic phosphate compound (silver sodium hydrogen zirconium phosphate; silver content of 3.8 percent), commercially available from Milliken Chemicals, was compared with the effect of silver nitrate. The silver containing antibacterial inorganic phosphate powder was added via dry blending with fumed silica at room temperature.

**Table 17: Addition of Silver Containing Inorganic Phosphate - CFU Counts for Various Polymers using a Mixture of Bacteria Strains Suspension (PM, EC, KP)**

| Sample | Time | CFU | Odor |
|---|---|---|---|
| Ex 15-0 Bacteria Strain Suspension (Mixture of PM, EC and KP)* | 0h | 197 | +++ |
| | 4h | 189 | |
| | 24h | 103.5 | |
| Control 15* | 0h | 132 | ++ |
| | 4h | 122 | |
| | 24h | 248 | |
| Ex 15-1* 0.13 percent ALPHASAN = 50 ppm Ag⁺ | 0h | 136.5 | 0 |
| | 4h | 1.6 | |
| | 24h | 0 | |
| Ex 15-2* 0.26 percent ALPHASAN = 100 ppm Ag⁺ | 0h | 119 | 0 |
| | 4h | 2.1 | |
| | 24h | 1 | |
| Ex 15-3* 0.53 percent ALPHASAN = 120 ppm Ag⁺ | 0h | 106 | 0 |
| | 4h | 0.8 | |
| | 24h | 0.3 | |
| Ex 15-4* 1.0 percent ALPHASAN = 380 ppm Ag⁺ | 0h | 91 | 0 |
| | 4h | 1 | |
| | 24h | 0.55 | |
| Ex 15-5 0.1890 g AgNO₃ = 100 ppm Ag⁺ | 0h | 92 | 0 |
| | 4h | 1.2 | |
| | 24h | 0 | |

Ex 15-4 treated with 1 percent ALPHASAN powder containing the calculated amount of 380 ppm of silver ions had a similar odor control effect as Ex 15-5 of the present invention containing only 100 ppm of silver ions, surprisingly indicating that less silver was needed if soluble silver salts according to the present invention were used.

### Experiment Series 16

The Sample Preparation Procedure was followed using 24 g of a natural zeolite (AGRICOLITE) in addition to AgNO₃, CuSO₄·5H₂O or ZnSO₄·7H2O. AGRICOLITE (Trademark of Agricola Metals Corporation, U.S.A.) is a potassium sodium aluminosilicate of the clinoptilolite type.

**Table 18: Polymers Treated with Silver Ions and Zeolite - CFU Counts for Various Polymers using a Mixture of Bacteria Strains Suspension (PM, EC, KP)**

| Sample | Time | CFU | Odor |
|---|---|---|---|
| Ex 16-0 Bacteria Strain Suspension (Mixture of PM, EC and KP)* | 0h | 79 | +++ |
| | 4h | 72 | |
| | 24h | 41.5 | |
| Control 16* | 0h | 50 | ++ |
| | 4h | 57.5 | |
| | 24h | 57 | |
| Ex 16-1* 12 g AGRICOLITE | 0h | 49.5 | + |
| | 4h | 64.5 | |
| | 24h | 75.5 | |
| Ex 16-2 12 g AGRICOLITE + 100 ppm Ag⁺= 0.1890 g AgNO₃ | 0h | 49.5 | 0 |
| | 4h | 66 | |
| | 24h | 0 | |
| Ex 16-3* 12 g AGRICOLITE + 0.25 percent Cu²⁺= 12 g CuSO₄·5H₂O | 0h | 54.5 | + |
| | 4h | 67 | |
| | 24h | 95.5 | |
| Ex 16-4* 12 g AGRICOLITE + 0.23 percent Zn²⁺ = 12 gZnSO₄·7H₂O | 0h | 36.5 | + |
| | 4h | 44.5 | |
| | 24h | 160 | |

The results indicate that the natural zeolite (AGRICOLITE) is not an effective odor control agent. The CFU results show that the polymer containing natural zeolite and silver ions (Ex 16-2) was effective for odor control, while copper and zinc ions in combination with natural zeolite did not show the positive effect on odor control.

### Experiment Series 17

In the following examples, silver ion exchanged zeolite was prepared and superabsorbent polymer particles were then treated with the zeolite.

AGRICOLITE zeolite material having a particle size distribution of from 0 to 100 µm was obtained by sieving AGRICOLITE zeolite material having a particle size distribution of from 0 to 0.5 mm. The zeolite (0 - 100 µm) was dried in an air-forced lab oven at 190°C for 3 h, and then cooled down to room temperature. 100.0 g of the dried zeolite were mixed with 300 g of deionized water in a 1 liter polyethylene bottle and agitated with a magnetic stirrer. 50 g of aqueous silver nitrate solution containing 1.00 g of silver nitrate were added to the zeolite and water slurry, and agitated for 16 h using a magnetic stirrer. The zeolite slurry was then filtered and the zeolite filter cake was dried in an air-forced oven for 3 hours at 190°C, and then cooled down to room temperature. The zeolite was further ground and sieved using a 100 µm sieve.

The Sample Preparation Procedure was followed using different amounts of silver ion exchanged AGRICOLITE.

**Table 19: Polymers Treated Silver Ion Exchanged Zeolites - CFU Counts for Various Polymers using a Mixture of Bacteria Strains Suspension (PM, EC, KP)**

| Sample | Time | CFU | Odor |
|---|---|---|---|
| Ex 17-0 Bacteria Strain Suspension (Mixture of PM, EC and KP)* | 0h | 111 | +++ |
| | 4h | 122 | |
| | 24h | 85 | |
| Control 17* | 0h | 120.5 | ++ |
| | 4h | 406 | |
| | 24h | 232 | |
| Ex 17-1* 0.42 percent AGRICOLITE = 26 ppm Ag⁺ | 0h | 94.5 | 0 |
| | 4h | 37.5 | |
| | 24h | 0.5 | |
| Ex 17-2* 0.83 percent AGRICOLITE = 53 ppm Ag⁺ | 0h | 132.5 | 0 |
| | 4h | 47.3 | |
| | 24h | 0 | |
| Ex 17-3* 1.67 percent AGRICOLITE = 106 ppm Ag⁺ | 0h | 37 | 0 |
| | 4h | 5.5 | |
| | 24h | 0 | |
| Ex 17-4* 2.5 percent AGRICOLITE = 159 ppm Ag⁺ | 0h | 83.5 | 0 |
| | 4h | 0 | |
| | 24h | 0 | |
| Ex 17-5 0.1890 g AgNO₃ = 100 ppm Ag⁺ | 0h | 68.5 | 0 |
| | 4h | 0 | |
| | 24h | 0 | |

2.5 percent silver ion exchanged zeolite containing a calculated amount of 159 ppm of silver ions had a similar odor control effect as Ex 17-5 of the invention containing only 100 ppm of silver ions, indicating that unexpectedly less silver was needed if soluble silver salts according to the present invention were used.

## Claims

1. A process for the preparation of water-absorbent, water-insoluble polymer particles, the process comprising:
(I) polymerizing a polymerization mixture comprising:
(a) one or more ethylenically unsaturated carboxyl-containing monomers,
(b) one or more crosslinking agents,
(c) optionally one or more comonomers copolymerizable with the carboxyl-containing monomer, and
(d) a polymerization medium,
to form a crosslinked hydrogel,
(II) comminuting the hydrogel to particles, and
(III) drying the hydrogel,
wherein a solution of a silver salt which has a solubility in water at room temperature of not less than 1 g per liter is added in at least one of the following steps:
(i) to the monomer mixture prior to the beginning of the polymerization or to the reaction mixture during polymerization, or
(ii) to the crosslinked hydrogel prior to or after comminution in step (II), or
(iii) to the dried polymer particles after step (III).

2. The process of claim 1 wherein the silver salt solution is added to the dried polymer particles after step (III).

3. The process of claims 1 or 2 further comprising step (IV) wherein the dried polymer particles from step (III) are heated to a temperature of from 170 to 250°C for from 1 to 60 minutes prior to or after addition of the silver salt.

4. The process of any of claims 1 to 3 wherein the silver salt is selected from the group consisting of silver nitrate, silver acetate, silver benzoate, silver bromate, silver chlorate, silver lactate, silver nitrite, silver perchlorate, silver permanganate, silver selenate, silver sulfate, and mixtures thereof.

5. The process of any of claims 1 to 4 wherein the silver salt is added in an amount providing 1 to 10,000 ppm silver cations, based on weight of dry polymer.

6. The process of claim 5 wherein the silver salt is added in an amount providing 100 to 1,000 ppm silver cations, based on weight of dry polymer.

7. The process of any of claims 1 to 6 wherein the solution of the silver salt is an aqueous solution.

8. The process of claim 7 wherein the aqueous solution of the silver salt additionally comprises a polyether polyol.

9. The process of any of claims 2 or 8 wherein the dried and optionally heat-treated polymer particles are treated with an aqueous solution of aluminum sulfate prior to, simultaneously with or after the addition of the silver salt solution.

10. The process of claims 2 to 8 further comprising addition of an additive to the dried and optionally heat-treated polymer particles prior to, simultaneously with or after the addition of the silver salt solution, wherein the additive is selected from the group consisting of activated carbon, chlorophillyn, chelating agents, soda, sodium bicarbonate, copper sulfate, copper acetate, zinc sulfate, silicates, silica, clay, cyclodextrin, citric acid, chitosan, ion exchange resin particles, zeolites or combinations thereof.

11. The process of claim 10 wherein fumed silica is mixed with the dried and optionally heat-treated polymer particles prior to or simultaneously with the addition of the silver salt solution.

12. The process of claim 11 wherein the fumed silica is employed as an aqueous dispersion.

13. Water-absorbent, water-insoluble polymer particles obtainable by the process of any of claims 1 to 12.

14. An absorbent structure comprising water-absorbent, water-insoluble polymer particles of claim 13 and at least one of a woven or nonwoven structure of paper, synthetic fibers, natural fibers, or a combination of these.

## Patentansprüche

1. Ein Verfahren zur Herstellung wasserabsorbierender, wasserunlöslicher Polymerpartikel, wobei das Verfahren umfasst:
(I) das Polymerisieren einer Polymerisationsmischung umfassend
(a) ein oder mehrere ethylenisch ungesättigte, Carboxylgruppen enthaltende Monomere,
(b) ein oder mehrere Vernetzungsmittel,
(c) gegebenenfalls ein oder mehrere, mit den Carboxylgruppen enthaltenden Monomeren copolymerisierbare Co-Monomere und
(d) ein Polymerisationsmedium, um ein vernetztes Hydrogel zu bilden,
(II) das Zerkleinern des Hydrogels unter Bildung von Partikeln und
(III) das Trocknen des Hydrogels,
wobei eine Lösung eines Silbersalzes mit einer Löslichkeit in Wasser bei Raumtemperatur von nicht weniger als 1 g pro Liter in mindestens einem der folgenden Schritte zugesetzt wird:
(i) zur Monomermischung vor dem Beginn der Polymerisation oder zur Reaktionsmischung während der Polymerisation, oder
(ii) zum vernetzten Hydrogel vor oder nach dem Zerkleinern in Schritt (II) oder
(iii) zu den getrockneten Polymerpartikeln nach Schritt (III) .

2. Das Verfahren nach Anspruch 1, wobei die Silbersalzlösung zu den getrockneten Polymerpartikeln nach Schritt (III) zugesetzt wird.

3. Das Verfahren nach Anspruch 1 oder 2, des Weiteren umfassend Schritt (IV), bei dem die getrockneten Polymerpartikel aus Schritt (III) auf eine Temperatur von 170 bis 250°C für 1 bis 60 Minuten vor oder nach Zugabe des Silbersalzes erhitzt werden.

4. Das Verfahren nach irgendeinem der Ansprüche 1 bis 3, wobei das Silbersalz ausgewählt ist aus der Gruppe bestehend aus Silbernitrat, Silberacetat, Silberbenzoat, Silberbromat, Silberchlorat, Silberlactat, Silbernitrit, Silberperchlorat, Silberpermanganat, Silberselenat, Silbersulfat und Mischungen daraus.

5. Das Verfahren nach irgendeinem der Ansprüche 1 bis 4, wobei das Silbersalz in einer Menge, welche 1 bis 10.000 ppm Silberkationen, bezogen auf das Gewicht des trockenen Polymers, bereitstellt, zugesetzt wird.

6. Das Verfahren nach Anspruch 5, wobei das Silbersalz in einer Menge, welche 100 bis 1.000 ppm Silberkationen, bezogen auf das Gewicht des trockenen Polymers, bereitstellt, zugesetzt wird.

7. Das Vefahren nach irgendeinem der Ansprüche 1 bis 6, wobei die Lösung des Silbersalzes eine wässrige Lösung ist.

8. Das Verfahren nach Anspruch 7, wobei die wässrige Lösung des Silbersalzes zusätzlich einen Polyetherpolyol umfasst.

9. Das Verfahren nach irgendeinem der Ansprüche 2 oder 8, wobei die getrockneten und gegebenenfalls hitzebehandelten Polymerpartikel mit einer wässrigen Aluminiumsulfat-Lösung vor, zeitgleich mit oder nach dem Zusetzen der Silbersalzlösung behandelt werden.

10. Das Verfahren nach den Ansprüchen 2 bis 8, des Weiteren umfassend das Zusetzen eines Additivs zu den getrockneten und gegebenenfalls hitzebehandelten Polymerpartikeln vor, zeitgleich mit oder nach dem Zusetzen der Silbersalzlösung, wobei das Additiv ausgewählt ist aus der Gruppe Aktivkohle, Chlorophillyn, Chelatisierungsmittel, Natriumcarbonat, Natriumbicarbonat, Kupfersulfat, Kupferacetat, Zinksulfat, Silikate, Silica, Ton, Cyclodextrin, Zitronensäure, Chitosan, Ionenaustausch-Harzpartikel, Zeolithe und Kombinationen daraus.

11. Das Verfahren nach Anspruch 10, wobei Siliziumdioxid (fumed silica)mit den getrockneten und gegebenenfalls hitzebehandelten Polymerpartikeln vor oder zeitgleich mit dem Zusetzen der Silbersalzlösung vermischt wird.

12. Das Verfahren nach Anspruch 11, wobei das Siliziumoxid (fumed silica) als eine wässrige Dispersion eingesetzt wird.

13. Wasserabsorbierende, wasserunlösliche Polymerpartikel, erhältlich durch das Verfahren nach irgendeinem der Ansprüche 1 bis 12.

14. Eine absorbierende Struktur umfassend wasserabsorbierende, wasserunlösliche Polymerpartikel nach Anspruch 13 und mindestens eine gewebte oder nicht gewebte Struktur aus Papier, synthetischen Fasern, natürlichen Fasern oder einer Kombination aus diesen.

## Revendications

1. Procédé de préparation de particules polymères hydroabsorbantes et insolubles dans l'eau, le procédé comprenant :
(I) la polymérisation d'un mélange de polymérisation comprenant :
(a) un ou plusieurs monomères éthyléniquement insaturés contenant un groupe carboxyle,
(b) un ou plusieurs agents de réticulation,
(c) éventuellement un ou plusieurs comonomères copolymérisables avec le monomère contenant un groupe carboxyle, et
(d) un milieu de polymérisation,
pour former un hydrogel réticulé,
(II) la réduction de l'hydrogel en particules, et
(III) le séchage de l'hydrogel,
une solution d'un sel d'argent qui a une solubilité dans l'eau à température ambiante supérieure ou égale à 1 g par litre étant ajoutée lors d'au moins une des étapes suivantes :
(i) au mélange de monomères avant le commencement de la polymérisation ou au mélange réactionnel au cours de la polymérisation, ou
(ii) à l'hydrogel réticulé avant ou après la réduction en particules de l'étape (II), ou
(iii) aux particules polymères séchées après l'étape (III).

2. Procédé selon la revendication 1 dans lequel la solution de sel d'argent est ajoutée aux particules polymères séchées après l'étape (III).

3. Procédé selon les revendications 1 ou 2 comprenant en outre l'étape (IV) dans laquelle les particules polymères séchées de l'étape (III) sont chauffées à une température comprise entre 170 et 250 °C pendant 1 à 60 minutes avant ou après l'ajout du sel d'argent.

4. Procédé selon l'une quelconque des revendications 1 à 3 dans lequel le sel d'argent est choisi dans le groupe constitué par le nitrate d'argent, l'acétate d'argent, le benzoate d'argent, le bromate d'argent, le chlorate d'argent, le lactate d'argent, le nitrite d'argent, le perchlorate d'argent, le permanganate d'argent, le sélénate d'argent, le sulfate d'argent, et les mélanges de ceux-ci.

5. Procédé selon l'une quelconque des revendications 1 à 4 dans lequel le sel d'argent est ajouté dans une quantité donnant de 1 à 10 000 ppm de cations d'argent, sur la base du poids du polymère sec.

6. Procédé selon la revendication 5 dans lequel le sel d'argent est ajouté dans une quantité donnant de 100 à 1000 ppm de cations d'argent, sur la base du poids du polymère sec.

7. Procédé selon l'une quelconque des revendications 1 à 6 dans lequel la solution du sel d'argent est une solution aqueuse.

8. Procédé selon la revendication 7 dans lequel la solution aqueuse du sel d'argent comprend en outre un polyéther-polyol.

9. Procédé selon l'une quelconque des revendications 2 ou 8 dans lequel les particules polymères séchées et éventuellement chauffées sont traitées avec une solution aqueuse de sulfate d'aluminium avant, en même temps que ou après l'ajout de la solution de sel d'argent.

10. Procédé selon les revendications 2 à 8 comprenant en outre l'ajout d'un additif aux particules polymères séchées et éventuellement chauffées avant, en même temps que ou après l'ajout de la solution de sel d'argent, l'additif étant choisi dans le groupe constitué par le charbon actif, la chlorophylline, les agents chélatants, la soude, le bicarbonate de sodium, le sulfate de cuivre, l'acétate de cuivre, le sulfate de zinc, les silicates, la silice, l'argile, la cyclodextrine, l'acide citrique, le chitosane, les particules de résine échangeuse d'ions, les zéolithes ou des combinaisons de ceux-ci.

11. Procédé selon la revendication 10 dans lequel de la silice sublimée est mélangée aux particules polymères séchées et éventuellement chauffées avant ou en même temps que l'ajout de la solution de sel d'argent.

12. Procédé selon la revendication 11 dans lequel la silice sublimée est utilisée sous la forme d'une dispersion aqueuse.

13. Particules polymères hydroabsorbantes et insolubles dans l'eau pouvant être obtenues par le procédé selon l'une quelconque des revendications 1 à 12.

14. Structure absorbante comprenant les particules polymères hydroabsorbantes et insolubles dans l'eau selon la revendication 13 et au moins un élément parmi une structure tissée ou non-tissée de papier, de fibres synthétiques, de fibres naturelles, ou une combinaison de celles-ci.
